# EUROPEAN PATENT APPLICATION

(11) **EP 4 039 475 A1**
(43) Date of publication of application: **10.08.2022**
(21) Application number: 21382088.9
(22) Date of filing: 03.02.2021
(51) Int. Cl.: B33Y 10/00, C12M 3/00, C12M 1/26

(54) **3D PRINTER TO GENERATE CURVED BIOLOGICAL TISSUE**

(71) Applicant: AJL Ophthalmic, S.A., 01510 Miñano (Alava) (ES)
(72) Inventor: NIETO, DANIEL, 15899 SANTIAGO DE COMPOSTELA (ES); GONZALEZ, MARIA, 01510 MIÑANO (ES); LARRA, EVA, 01510 MIÑANO (ES)
(74) Representative: Fernandez Pou, Felipe

(57) **Abstract**

3D printer to generate curved biological tissue that comprises: a structure (2) having a moving head (3) by means of guides (4, 4') for the travel on the three axis XYZ on the space regulating its positioning on a supporting base (5) where the material is deposited; a transparent mold device, composed of a transparent curved-convex glass (6) coupled below the moving head (3) and of a transparent curved-concave glass (7), complementary to the convex glass (6), that is incorporated under the head (3) on the supporting base (5) where the material is deposited; and a UV light irradiation optical device (8); so that, when the biomaterial has being deposited on the curved-concave glass (7), the approach of the curved-convex glass (6) to it and the further UV light irradiation, generate the layers of the tissue to be formed having the specific shape defined by the said mold.

## Description

### OBJECT OF THE INVENTION

The invention, as stated in the title of this specification, refers to a 3D printer to generate curved biological tissue that provides the function to which it is designed with advantages and characteristics, that are disclosed in detail thereafter.

The object of this invention refers to a 3D printer specially designed to allow generating curved biological tissue, namely corneal tissue, by projecting layer by layer the different materials that have to constitute the corneal tissue on a hemispherical transparent mold, having the necessary radius of curvature, and the related process of UV light curing of each layer of the cornea to be developed.

### FIELD OF APPLICATION OF THE INVENTION

The field of application of this invention is within the sector of industry engaged in manufacturing 3D printing devices, apparatuses and systems, focusing namely in the field of printing with biological material.

### BACKGROUND OF THE INVENTION

Printing with biological material or bioprinting is a disruptive activity and quickly spreading in the field of organs and tissues engineering [1] that can be described as the automated creation of tissues or organs tissues through the operation of additive and subtractive manufacturing processes. The challenge is to create an anatomically accurate structure, in which the living cells can be incorporated to the biomaterials, with suitable biochemical signalling agents, so that the construction of assembled tissue can be self-organized and be kept after its manufacture.

The potential of the bioprinting when producing transplantable tissue structures is making progress because new materials and processes appear for the multilayer skin [2], vascular grafts [3], nerve grafts [4], trachea [5], muscles [6], bone [7], cartilage [8] and heart tissue [9].

In addition, the bioprinting is becoming a powerful tool to produce high level tissue models for the research and administration of pharmaceutical drugs [10-13].

The recent advances in the methods to produce additives in 3D, such as extrusion, laser bioprinting, stereolithography and bioprinting based on nozzles, allowed that the researchers manufacture complex structures having controlled architectures and properties [14, 15].

Later, the interest to implementing these 3D technologies in tissues engineering is growing. For example, the 3D tissue structures having the capacity to simulate the physiology and complex functionalities of the tissue have an unbelievable potential for their use to generating artificial tissues.

However, there still exist several challenges to have to be reached.

We are concretely speaking of:
- generating the vasculature associated to each type of tissue,
- generating multi-material tissues having several cellular types
- and generating different morphologies having properties associated to each tissue.

In the case of the cornea: curved, multi-material and transparent tissue.

In this sense, the objective of this invention is to develop a 3D stereolithographic bioprinting machine to generate artificial, complex and multi-material tissues such as the corneal tissue.

On the other hand, and as reference to the current state-of-the-art, although in the market different types of 3D printers are known, at least the applicant, is not aware of the existence of any that possesses technical and structural characteristics same or similar to those of the invention claimed herein.

In this sense, it shall be pointed out that, as the document closest to the object of this invention, the patent EP3398778A1 is known that discloses a 3D printer capable of dispensing biological material (including materials comprising living cells) composed by a spray head, a connector and a container of bioprinting material that possesses a discharge pipe, in which a first end of the connector is connected screwed with the discharge pipe and a second end of the connector is removably connected to the spray head. The said printer, however, is significantly different from that which is herein proposed in multiple technical aspects, namely those referring to the transparent mold and UV light curing process that precisely are those characterizing the printer of this invention and that allow its main application to forming corneal tissue, having a curved configuration and formed by different types of material, transparent material included.

### EXPLANATION OF THE INVENTION

The 3D printer to generate curved biological tissue that the invention proposes is configured as the suitable solution to the above-mentioned objective, the characterizing details making it possible and that distinguish it conveniently appearing in the final claims attached to this description.

Concretely, what the invention proposes, as it was stated above, is a 3D printer that possesses the feature of being specially designed to allow generating curved biological tissue, namely corneal tissue, by projecting layer by layer the different materials that have to constitute the corneal tissue on a hemispherical transparent mold, having the necessary radius of the curvature, and the related process of UV light-curing each layer of the cornea to be developed.

Thus, the main innovation of the printer of the invention is based on the application of photonics concepts in combination with microfluidic systems and customized heads for the requirements of biofabrication of each customer in particular.

The main challenges associated to the 3D biofabrication of tissues include their simplified structures and their limited potential of vascularization. The challenges associated to the existing techniques of biofabrication have prevented to generate in vitro functional models that keep their characteristics in vivo which are prerequisites for accurate biological studies. To overcome these limitations, the printer of the invention comprises a DMD *(Digital Mirror Device)* or transparent mold composed of a curved-convex glass coupled to the printer moving head and a curved-concave glass incorporated on a material receiving support, which in combination with an optical system of UV light irradiation and of delivery of biomaterials charged with cells, allows to reach resolutions of 10µm with biofabrication speeds relatively quick to keep the cell viability.

Likewise, the printer of the invention is a modular stereolithographic 3D printer type with a system of multi-material exchangeable heads that allows the possibility to obtain resolutions of 1-10µm, as well as to generate curved tissues adapted to different curvature morphologies by means of the said heads customization, based on the morphologic needs in each particular case.

With all that, the printer of the invention constitutes a tissues biofabrication system having a curvature, by means of the adaptation of the transparent mold of the head to use different biomaterials having characteristics compatible with corneal tissue, that means, it allows to generate tissue having controlled curvatures.

In addition, the printer allows to control and adjust the conditions of the parameters of biofabrication, that means, temperature, speed, times of UV exposure, cell viability, according to the characteristics of viscosity and properties of the components of the bioink that to such aim will have to be added to the biomaterial.

Thus, preferably, the printer contemplates the use of bioinks to be used as extracellular matrix for the biofabrication of the corneal tissue. The objective is to obtain photosensitive bioinks (GelMa, PEGDA) out of different biomaterials (collagen I, hyaluronic acid, jelly, agarose, fibrin, GelMA and fibronectin, etc.) capable to keep the cells viable without altering the physical-chemical characteristics:
- The composition of the bioinks will be modified to increase their gelation by mixing in different proportions biocompatible hydrogels. Methacrylate groups (MA) will be added, to confer capacity of photo reticulation and a photo initiator sensitive to the bioprinter wavelength. Several Collagen/Agarose concentrations were tried.

The agarose will provide structural support to the collagen.
- The materials initially selected for developing the biomaterial are: GelMa, Agarose-collagen I

For all this, in the area of the printer head it is contemplated to include the mold disclosed that is composed of an upper transparent glass having a predefined curved-concave curvature, arranged so that it remains located below the moving head. And a lower hemispherical transparent glass, located on a support under the moving head, having a concave curvature that fit in the curvature of the upper glass, so that the gap between the two glasses will provide the thickness of each layer of tissue to be manufactured.

This head allows to work with two configurations, with UV curing and by means of the temperature.

Optionally, the printer contemplates a microfluidics system to fill with biomaterial the lower glass of the head area that, although it can be made by hand, for example through an injection, alternativelly, it is contemplated

to integrate a biomaterial pumping system by means of the integration in the head of an inlet channel and another of outlet.

### DESCRIPTION OF THE DRAWINGS

To complement the description that is carried out and in order to assist to a best understanding of the characteristics of the invention, attached to this specification, as an integral part thereof, there is a drawing in which for illustration and no limiting purpose the following has been represented:

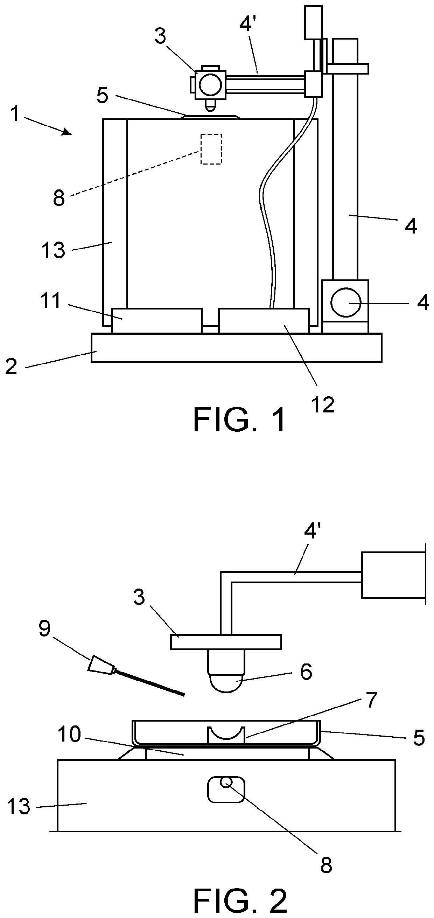

The figure number 1.- It shows a schematic elevation view of an example of embodiment of the 3D printer to generate curved biological tissue object of the invention, the main parts and elements that it comprises can be seen; and

The figure number 2.- It shows a schematic section view of the area of the head of the 3D printer of the invention, the configuration and arrangement of the main elements comprised in the said area can be seen.

### PREFERRED EMBODIMENT OF THE INVENTION

Seen the said figures, and according to the numerals adopted in them, a non-limiting example of embodiment can be seen of the 3D printer to generate curved biological tissue of the invention, which comprises what is described in detail thereafter.

Thus, as it can be observed in the said figures, the printer (1) 3D of the invention, comprises, in a well-known manner, a structure (2) having a moving head (3) by means of a guides mechanism (4, 4') for its travel on the three axis XYZ of the space (X longitudinal, Y vertical, Z lateral) to regulate its positioning on a supporting base (5) on which the material is being deposited.

And, from this configuration, the printer (1) of the invention is distinguished, essentially, because it comprises:
- a transparent mold device, composed of a transparent curved-convex glass (6) coupled below the moving head (3) and a transparent curved-concave glass (7), complementary to the convex glass (6), that is incorporated under the head (3) on the supporting base (5) where the material is deposited, and
- an optical device for UV light (8) irradiation,
so that, when an amount of biomaterial has been successively deposited on the curved-concave glass (7), the approach of the curved-convex glass (6) on the curved-concave glass (7) and the further UV light irradiation, generates each of the layers of tissue to be formed with the specific shape defined by the said mold.

In an embodiment, depositing biomaterial on the curved-concave glass (7) is carried out by manual means of application of material (9).

Alternatively, the printer (1) comprises, as means of application of material (9), a pumping system integrated in the head (3) having an inlet channel and another of outlet. Although this system has not been represented in the figures.

Preferably, the moving head (3) and/or, at least, the curved-convex glass (6) it incorporates, as well as the curved-concave glass (7) of the support (5) are easily removable and exchangeable to incorporate, in each case, a curved-convex glass (6) and a curved-concave glass (7) having the respective radius of internal and external curvature suitable depending on the morphology that is sought of the corneal tissue to generate.

Preferably, the UV light irradiation optical device (8) comprises, at least, a UV led lamp incorporated in its interior.

Preferably, the supporting base (5) is a transparent removable container that is incorporated on a hollow (10) provided on the upper part of the housing (13) of the UV optical device (8)

Preferably the UV optical device (8) is connected to an intensity electronic controller (11).

Preferably, the guides mechanism (4) for the travel of the head (3) on the XY axis (longitudinal and vertical) is hand-operated, while the guides mechanism (4') for the travel of the head (3) on the Z axis (lateral) is a power-operated operating mechanism by means of a high-resolution electronic controller (12). Concretely with capacity of control of movement up to 20nm.

Sufficiently described the nature of this invention, as well as the manner to implement it, it is not deemed necessary to extend any longer its explanation in order that any person skilled in the art understands the extent and advantages derived from it.

## Claims

1. 3D printer to generate curved biological tissue that, comprising a structure (2) having a moving head (3) by means of a guides mechanism (4, 4') for its travel on the three axis XYZ of the space to regulate its positioning on a supporting base (5) on which the material is being deposited, is **characterized in that it** comprises: -a transparent mold device, composed of a transparent curved-convex glass (6) coupled below the moving head (3) and of a transparent curved-concave glass (7) complementary to the convex glass (6), that is incorporated under the head (3) on the supporting base (5) where the material is deposited, and - a UV light irradiation optical device (8), So that, when successively an amount of biomaterial has been deposited on the curved-concave glass (7), the approach of the curved-convex glass (6) on the curved-concave glass (7) and the further UV light irradiation, generate each of the layers of the tissue to be formed with the concrete shape defined by the said mold.

2. 3D printer to generate curved biological tissue, according to the claim 1, **characterized in that it** comprises, as material application means (9), a pumping system integrated in the head (3) having an input channel and another of output.

3. 3D printer to generate curved biological tissue, according to the claim 1 or 2, **characterized in that** the curved-convex glass (6) and the curved-concave glass (7) removable and exchangeable to incorporate, in each case a curved-convex glass (6) and a curved-concave glass (7) having the respective radius of internal and external curvature that is suitable depending on the morphology sought of the corneal tissue to be generated.

4. 3D printer to generate curved biological tissue, according to any of the claims 1 a 3, **characterized in that** the optical device of UV light irradiation (8) comprises, at least, a UV led lamp incorporated in its interior.

5. 3D printer to generate de curved biological tissue, according to any of the preceding claims, **characterized in that** the supporting base (5) is a removable transparent container that is incorporated on a hollow (10) provided on the upper part of the housing of the UV optical device (8).

6. 3D printer to generate curved biological tissue, according to any of the preceding claims, **characterized in that** the UV optical device (8) is connected to an intensity electronic controller (11).

7. 3D printer to generate curved biological tissue, according to any of the preceding claims, **characterized in that** the guides mechanism (4') for the travel head (3) on the Z axis is a power-operated operating mechanism d by means of a high-resolution electronic controller (12).

8. 3D printer to generate curved biological tissue, according to the claim 7, **characterized in that** the guides mechanism (4') for the travel of the head (3) on the Z axis is a power-operated mechanism by means of an electronic controller having the capacity to control movement up to 20nm.
